# EUROPEAN PATENT APPLICATION

(11) **EP 4 729 089 A1**
(43) Date of publication of application: **22.04.2026**
(21) Application number: 24822311.7
(22) Date of filing: 14.03.2024
(51) Int. Cl.: A61M 5/142

(54) **LIQUID MEDICINE SUPPLY DEVICE AND USE METHOD THEREFOR**

(30) Priority: 16.06.2023 CN 202310717390
(71) Applicant: YW MEMS (SUZHOU) CO., LTD, Suzhou, Jiangsu 215000 (CN)
(72) Inventor: MA, Shuo, Suzhou, Jiangsu 215000 (CN); HU, Huishan, Suzhou, Jiangsu 215000 (CN); SONG, Qingyang, Suzhou, Jiangsu 215000 (CN)
(74) Representative: Bryers Intellectual Property Ltd
(86) International application number: PCT/CN2024/081598
(87) International publication number: WO 2024/255353

(57) **Abstract**

Disclosed in the present application are a liquid medicine supply device and a use method therefor. The liquid medicine supply device comprises an injection tube, a first plunger, a liquid storage bag and a positive displacement pump. The injection tube is provided with a first end and a second end, which are opposite each other, wherein the first end is provided with a medicine feeding port and a medicine output port, which is configured to connect to an infusion tube; a limiting structure for stopping the plunger is provided at a position close to the second end inside the injection tube; and a sealable first exhaust port is provided in a first tube section between the limiting structure and the second end. The first plunger is arranged in a second tube section between the limiting structure and the first end; the liquid storage bag is used for storing a power fluid; and a liquid inlet of the positive displacement pump is connected to the liquid storage bag by means of tubing, and a liquid outlet of the positive displacement pump is connected to the first tube section by means of tubing. The liquid medicine supply device provided in the present application has a simple structure, thereby reducing the production and manufacturing costs; the phenomenon of the agglomeration of substances in a liquid medicine occurring during the delivery of the liquid medicine can be prevented, thereby reducing the impact of the delivery process on the efficacy of the medicine; in addition, the liquid medicine supply device also helps to improve the control precision for a medicine dose.

## Description

This application claims priority to Chinese Patent Application No. 202310717390.3, filed with the China National Intellectual Property Administration on June 16, 2023, and entitled "LIQUID MEDICINE SUPPLY DEVICE AND USE METHOD THEREFOR", the entire content of which is incorporated herein by reference.

### TECHNICAL FIELD

The present application relates to the technical field of medical devices, and in particular to a liquid medicine supply device and a use method therefor.

### BACKGROUND

In many scenarios, liquid medicine supply devices are needed, for example, for the timed and quantitative delivery of insulin to diabetic patients, and the timed and quantitative delivery of analgesics to post-operative patients. Currently, a common liquid medicine supply device pumps liquid medicine into a patient's body through a volumetric pump. Although this can simplify the structure and reduce costs compared to traditional liquid medicine supply devices, there are still certain deficiencies: the liquid medicine passes through the volumetric pump and undergoes a suction and pumping process. This process often causes substances in the liquid medicine, such as active ingredient proteins and polypeptides, to experience an aggregation phenomenon, which in turn adversely affects the efficacy of the medicine. Moreover, the components of liquid medicines vary, and different liquid medicines may have significant differences in viscosity. In order to achieve fine control of the dosage, the pumping accuracy of the volumetric pump has to be calibrated for the liquid medicine, causing difficulties in equipment production or use. Therefore, how to improve the liquid medicine supply device to overcome the above deficiencies has become a technical problem that needs to be urgently solved by those skilled in the art.

### SUMMARY

In one aspect, the present application provides the following technical solution:
A liquid medicine supply device, comprising:
an injection tube having a first end and a second end opposite to each other, wherein the first end is provided with a medication inlet and a medication outlet, the medication outlet is for connecting to an infusion tube, a limiting structure for stopping a piston is provided inside the injection tube at a position close to the second end, and a first tube section between the limiting structure and the second end is provided with a first exhaust port closable;
a first piston provided within a second tube section, the second tube section is between the limiting structure and the first end;
a liquid storage bag configured to store a power fluid; and
a volumetric pump, wherein a liquid inlet of the volumetric pump is connected to the liquid storage bag through a pipeline, and a liquid outlet of the volumetric pump is connected to the first tube section through a pipeline.

Optionally, the above liquid medicine supply device further comprises a pressure sensor provided within the first tube section.

Optionally, in the above liquid medicine supply device, the volumetric pump is a diaphragm pump.

Optionally, in the above liquid medicine supply device, the limiting structure is an annular rib provided on an inner wall of the injection tube.

Optionally, the above liquid medicine supply device comprises a controller electrically connected to the volumetric pump, and the controller is provided with a timing module.

Optionally, in the above liquid medicine supply device, a sealing member for closing the first exhaust port is provided at the first exhaust port, and the sealing member is made of a material capable of absorbing the power fluid and swelling.

Optionally, in the above liquid medicine supply device, a hose is connected to the medication outlet, an electronically controlled liquid stopper or a liquid stopping clamp manually controlled is provided at an end of the hose close to the liquid outlet, and a connector for connecting to the infusion tube is provided at an end of the hose away from the liquid outlet.

Optionally, the above liquid medicine supply device further comprises a second piston provided within the second tube section, the second piston acts located on a side of the first piston close to the medication outlet, the second tube section is provided with a second exhaust port closable, and a distance from the second exhaust port to the limiting structure matches a thickness of the first piston.

Optionally, the above liquid medicine supply device further comprises a temporary stop member arranged with the second tube section, and a distance from the temporary stop member to the limiting structure matches the thickness of the first piston; wherein the temporary stop member at least has a first state and a second state, wherein in the first state, the temporary stop member protrudes from the inner wall of the injection tube, and in the second state, the temporary stop member does not protrude from the inner wall of the injection tube.

A use method for the above liquid medicine supply device, wherein the use method comprises:
Step A, adding liquid medicine: in a state when the medication outlet is closed and the first exhaust port is open, injecting liquid medicine into the injection tube through the medication inlet, causing the first piston originally located at the first end to move toward aposition of the limiting structure under the pushing of the liquid medicine, and closing the medication inlet;
Step B, adding power fluid: in a state when the first exhaust port faces upward, injecting a portion of the power fluid in the liquid storage bag into the injection tube by the volumetric pump, causing the first tube section to be filled with the power fluid, and closing the first exhaust port; and
Step C, pushing liquid medicine: opening the medication outlet, and pumping the power fluid to the injection tube by the volumetric pump, causing the first piston to move toward the first end under the pushing of the power fluid, thereby pushing the liquid medicine in the injection tube to the infusion tube.

A use method for the above liquid medicine supply device, wherein the use method comprises:
Step A, adding power fluid: switching the temporary stop member to the first state to stick the first piston originally located at the limiting structure, and in a state when the first exhaust port faces upward and is open, injecting a portion of the power fluid in the liquid storage bag into the injection tube by the volumetric pump, causing the first tube section to be filled with the power fluid, and closing the first exhaust port;
Step B, adding liquid medicine: switching the temporary stop member to the second state, and in a state when the medication outlet is closed and the second exhaust port is open, injecting liquid medicine into the injection tube through the medication inlet, causing the second piston originally located at the first end to move toward the second end under the pushing of the liquid medicine, until the second piston is in tight contact with the first piston, and closing the medication inlet and the second exhaust port; and
Step C, pushing liquid medicine: opening the medication outlet, and pumping the power fluid to the injection tube by the volumetric pump, causing the first piston and the second piston to move toward the first end under the pushing of the power fluid, thereby pushing the liquid medicine in the injection tube to the infusion tube.

The liquid medicine supply device provided by the present application has the following beneficial effects:
After the volumetric pump pumps the power fluid in the liquid storage bag into the injection tube, the power fluid is used to push the piston in the injection tube to move, thereby realizing the delivery of the liquid medicine in the injection tube. Such a configuration makes the overall structure of the liquid medicine supply device relatively simple and reduces production and manufacturing costs. Moreover, the liquid medicine does not flow through the volumetric pump, avoiding the aggregation phenomenon of substances in the liquid medicine during liquid medicine delivery, thereby reducing the impact of the delivery process on the efficacy of the medicine. In addition, even if the liquid medicine to be supplied is different, the volumetric pump still pumps the same power fluid, so the pumping accuracy of the volumetric pump is not affected, which is conducive to improving the control accuracy of the dosage.

### BRIEF DESCRIPTION OF THE DRAWINGS

In order to more clearly illustrate the technical solutions in the embodiments of the present application or in the prior art, the drawings required for the description of the embodiments or the prior art will be briefly introduced below. Obviously, the drawings in the following description are only embodiments of the present application, and for those of ordinary skill in the art, other drawings can also be obtained according to the provided drawings without creative efforts.
FIG. 1 is a schematic diagram of a liquid medicine supply device provided by Embodiment 1 of the present application;
FIG. 2 is a schematic diagram of a liquid medicine supply device provided by Embodiment 2 of the present application; and
FIG. 3 is a schematic diagram of a liquid medicine supply device provided by Embodiment 3 of the present application.

### Reference numerals in the figures:

1. Injection tube; 11. Medication inlet; 12. Medication outlet; 13. Limiting structure; 14. First exhaust port; 15. Second exhaust port; 16. Temporary stop member; 21. First piston; 22. Second piston; 3. Pressure sensor; 4. Volumetric pump; 41. Volume change driver; 42. Liquid outlet; 43. Liquid inlet; 5. Liquid storage bag; 51. Liquid filling port.

### DETAILED DESCRIPTION

The technical solutions in the embodiments of the present application will be described clearly and completely below with reference to the accompanying drawings in the embodiments of the present application. Obviously, the described embodiments are only a part of the embodiments of the present application, rather than all of the embodiments. Based on the embodiments in the present application, all other embodiments obtained by those of ordinary skill in the art without creative efforts shall fall within the protection scope of the present application.

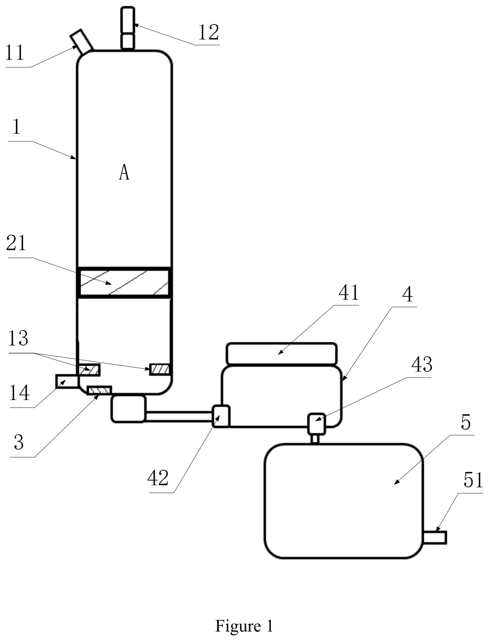

Referring to FIG. 1, Embodiment 1 of the present application provides a liquid medicine supply device, comprising an injection tube 1, a first piston 21, a liquid storage bag 5, and a volumetric pump 4. The injection tube 1 has a first end and a second end opposite to each other. The first end is provided with a medication inlet 11 and a medication outlet 12, the medication outlet is for connecting to an infusion tube. A limiting structure 13 for stopping a piston is provided inside the injection tube 1 at a position close to the second end. A first tube section between the limiting structure 13 and the second end is provided with a first exhaust port 14 closable. The first piston 21 is provided within a second tube section, the second tube section is between the limiting structure 13 and the first end; that is, the injection tube 1 is divided into the first tube section close to the second end and the second tube section close to the first end by the limiting structure 13, and the first piston 21 can only move within the second tube section. The liquid storage bag 5 is used for storing power fluid. A liquid inlet 43 of the volumetric pump 4 is connected to the liquid storage bag 5 through a pipeline, and a liquid outlet 42 of the volumetric pump 4 is connected to the first tube section through a pipeline.

Based on the liquid medicine supply device provided by Embodiment 1, the present application provides a use method for the liquid medicine supply device, and the use method comprises the following steps:
S11, adding liquid medicine: in a state when the medication outlet 12 is closed and the first exhaust port 14 is open, liquid medicine is injected into the injection tube 1 through the medication inlet 11, causing the first piston 21 originally located at the first end to move toward a position of the limiting structure 13 under the pushing of the liquid medicine, and closing the medication inlet 11.

The first piston 21 originally being at the first end means that in the factory state of the liquid medicine supply device, the volume of cavity A in FIG. 1 is zero. Therefore, as the liquid medicine is continuously injected, the volume of cavity A continuously increases, and the gas on the other side of the first piston 21 is gradually discharged from the first exhaust port 14. When the first piston 21 arrives at the position of the limiting structure 13, due to the blocking effect of the limiting structure 13, the first piston 21 cannot continue to move. At this time, the volume of cavity A reaches a maximum value, and cavity A is filled with liquid medicine. The volume of cavity A being zero before adding liquid medicine can ensure that the liquid medicine added to the injection tube will not accumulate air bubbles.

S12, adding power fluid: in a state when the first exhaust port 14 faces upward, a portion of the power fluid in the liquid storage bag 5 is injected into the injection tube 1 by the volumetric pump 4, causing the first tube section to be filled with the power fluid, and closing the first exhaust port 14.

In the factory state of the liquid medicine supply device, the liquid storage bag 5 is already filled with power fluid, and the amount of power fluid is sufficient for the use in Step S12 and the subsequent Step S13. In the aforementioned Step S11, the first piston 21 stops moving upon reaching the limiting structure 13, so the gas in the injection tube 1 is not completely discharged. The remaining gas is discharged through Step S12; that is, as the power fluid is continuously injected, the power fluid gradually fills the cavity on the side of the first piston 21 away from the liquid outlet 42.

S13, pushing liquid medicine: the medication outlet 12 is opened, and power fluid is pumped to the injection tube 1 by the volumetric pump 4, causing the first piston 21 to move toward the first end under the pushing of the power fluid, thereby pushing the liquid medicine in the injection tube 1 to the infusion tube.

Since the cavities on both sides of the first piston 21 are filled with liquid medicine and power fluid respectively, in Step S13, the amount of power fluid pumped into the injection tube 1 by the volumetric pump 4 is equal to the amount of liquid medicine flowing out of the medication outlet 12. Since the liquid medicine does not flow through the volumetric pump 4, the aggregation phenomenon of substances in the liquid medicine, such as active ingredient proteins and polypeptides, caused by the suction and pumping process is avoided, which is conducive to ensuring that the efficacy of the medicine is not affected. Moreover, even if the liquid medicine to be supplied is different, the volumetric pump 4 still pumps the same power fluid, ensuring that the pumping accuracy of the volumetric pump 4 is not affected.

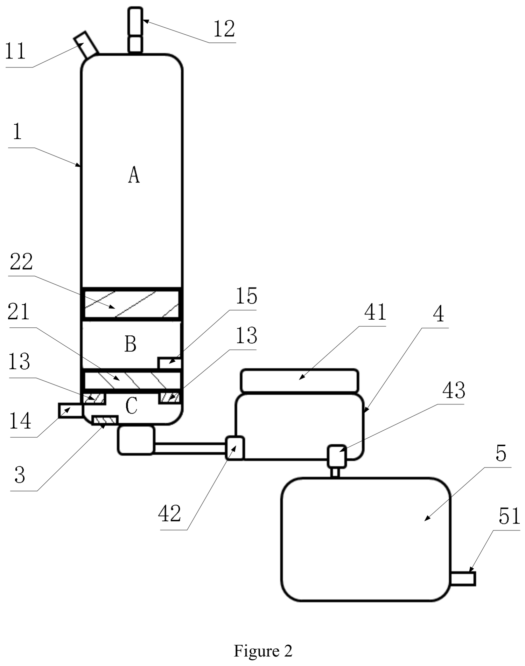

Referring to FIG. 2, Embodiment 2 of the present application provides a liquid medicine supply device. The difference from Embodiment 1 is that Embodiment 2 further comprises a second piston 22 provided within the second tube section. The second piston 22 is located on a side of the first piston 21 close to the medication outlet 12. Moreover, the second tube section is provided with a second exhaust port 15 closable, and the distance from the second exhaust port 15 to the limiting structure 13 matches the thickness of the first piston 21; that is, when the first piston 21 abuts against the limiting structure 13, a surface of the first piston 21 away from the limiting structure 13 is flush with the second exhaust port 15, so that the gas between the first piston 21 and the second piston 22 can be completely discharged from the second exhaust port 15.

Based on the liquid medicine supply device provided by Embodiment 2, the present application provides a use method for the liquid medicine supply device, and the use method comprises the following steps:
S21, adding liquid medicine: in a state when the medication outlet 12 is closed and the second exhaust port 15 is open, liquid medicine is injected into the injection tube 1 through the medication inlet 11, causing the second piston 22 originally located at the first end to move toward the second end under the pushing of the liquid medicine, until the second piston 22 is in tight contact with the first piston 21 originally located at the limiting structure 13, and closing the medication inlet 11 and the second exhaust port 15.

The first piston 21 originally being located at the limiting structure 13 means that in the factory state of the liquid medicine supply device, the first piston 21 stays between the second exhaust port 15 and the limiting structure 13 as shown in FIG. 2. Therefore, in Step S21 and the subsequent Step S22, the volume of cavity C in FIG. 2 does not change.

The second piston 22 originally being at the first end means that in the factory state of the liquid medicine supply device, the volume of cavity A in FIG. 2 is zero. Therefore, as the liquid medicine is continuously injected, the volume of cavity A continuously increases, and the gas in cavity B is gradually discharged from the second exhaust port 15. The volume of cavity A being zero before adding liquid medicine can ensure that the liquid medicine added to the injection tube will not accumulate air bubbles. When the second piston 22 arrives at a position of the first piston 21, it is blocked and cannot continue to move. At this time, the volume of cavity A reaches a maximum value, and the volume of cavity B becomes zero; that is, the second piston 22 is in tight contact with the first piston 21.

S22, adding power fluid: in a state when the first exhaust port 14 faces upward and is open, a portion of the power fluid in the liquid storage bag 5 is injected into the injection tube 1 by the volumetric pump 4, causing the first tube section to be filled with the power fluid, and closing the first exhaust port 14.

In the factory state of the liquid medicine supply device, the liquid storage bag 5 is already filled with power fluid, and the amount of power fluid is sufficient for the use in Step S22 and the subsequent Step S23. Through Step S22, the gas in cavity C in FIG. 2 can be completely discharged; that is, as the power fluid is continuously injected, the gas in cavity C is discharged from the first exhaust port 14 until the power fluid fills cavity C.

S23, pushing liquid medicine: opening the medication outlet 12, and power fluid is pumped to the injection tube 1 by the volumetric pump 4, causing the first piston 21 and the second piston 22 to move toward the first end under the pushing of the power fluid, thereby pushing the liquid medicine in the injection tube 1 to the infusion tube.

Since cavity B has disappeared, and cavity A and cavity C are filled with liquid medicine and power fluid respectively, in Step S23, the amount of power fluid pumped into the injection tube 1 by the volumetric pump 4 is equal to the amount of liquid medicine flowing out of the medication outlet 12.

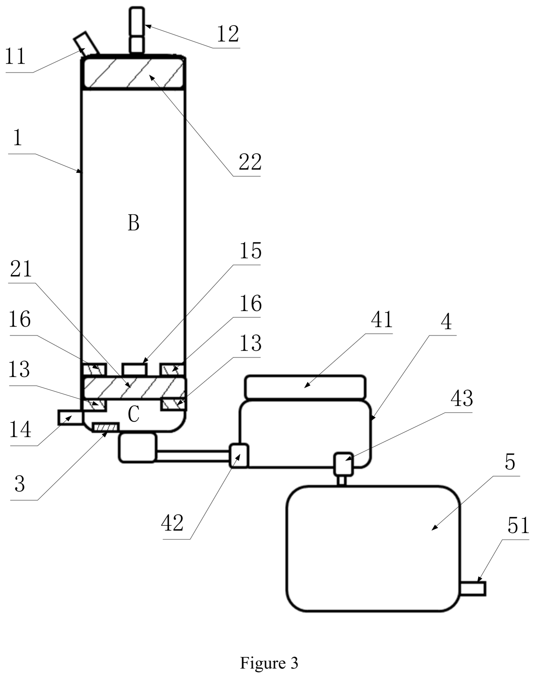

In addition, in order to further simplify the use steps of the liquid medicine supply device, the step of adding power fluid can be completed in advance during the manufacture of the liquid medicine supply device provided by Embodiment 2; that is, in the factory state, cavity C is already filled with power fluid. In this way, in the subsequent use process, only the two steps of adding liquid medicine and pushing liquid medicine described above need to be performed. As shown in FIG. 2, the liquid storage bag 5 can be provided with a liquid filling port 51. Thus, during the manufacture of the liquid medicine supply device, power fluid is continuously injected through the liquid filling port 51 to ensure that the liquid storage bag 5 is always in a maximum volume state, and after the power fluid fills cavity C, the first exhaust port 14 and the liquid filling port 51 are closed. It should be noted that in the above process, the air pressure in cavity B should satisfy the condition that the first piston 21 does not move when adding power fluid. Of course, in addition to the method of using the air pressure in cavity B to prevent the first piston 21 from moving in the above process, a limiting member functioning as a temporary stop can also be used to prevent the first piston 21 from moving in the above process. For example, FIG. 3 shows a liquid medicine supply device provided by Embodiment 3 of the present application. The difference from Embodiment 2 is that Embodiment 3 further comprises a temporary stop member 16 arranged with the second tube section. The distance from the temporary stop member 16 to the limiting structure 13 matches the thickness of the first piston 21; that is, the temporary stop member 16 and the second exhaust port 15 are located at the same position in the axial direction of the injection tube 1. Moreover, the temporary stop member 16 at least has a first state and a second state. In the first state, the temporary stop member 16 protrudes from the inner wall of the injection tube 1, and in the second state, the temporary stop member 16 does not protrude from the inner wall of the injection tube 1. When manufacturing the liquid medicine supply device, the temporary stop member 16 is first switched to the first state, so that the first piston 21 is stuck between the temporary stop member 16 and the limiting structure 13. In this way, the step of adding power fluid can be performed to fill cavity C with power fluid. After closing the first exhaust port 14, the temporary stop member 16 is switched to the second state; that is, in the factory state of the liquid medicine supply device, the temporary stop member 16 retracts from the inner cavity of the injection tube 1 to avoid affecting the subsequent movement of the piston.

When cavity C in the factory state of the liquid medicine supply device contains air, that is, when the producer has not injected power fluid into cavity C, the user of the liquid medicine supply device can also operate the aforementioned temporary stop member 16 to complete the step of adding power fluid. For example, based on the liquid medicine supply device provided by Embodiment 3, the present application provides a use method for the liquid medicine supply device, and the use method comprises the following steps:
S31, adding power fluid: switching the temporary stop member 16 to the first state to stick the first piston 21 originally located at the limiting structure 13, and in a state when the first exhaust port 14 faces upward and is open, injecting a portion of the power fluid in the liquid storage bag 5 into the injection tube 1 by the volumetric pump 4, causing the first tube section to be filled with the power fluid, and closing the first exhaust port 14.

The first piston 21 originally being located at the limiting structure 13 means that in the factory state of the liquid medicine supply device, the first piston 21 stays between the second exhaust port 15 and the limiting structure 13 as shown in FIG. 3. Therefore, in Step S31 and the subsequent Step S32, the volume of cavity C in FIG. 3 does not change.

In the factory state of the liquid medicine supply device, the liquid storage bag 5 is already filled with power fluid, and the amount of power fluid is sufficient for the use in Step S31 and the subsequent Step S33. Through Step S31, the gas in cavity C in FIG. 3 can be completely discharged; that is, as the power fluid is continuously injected, the gas in cavity C is discharged from the first exhaust port 14 until the power fluid fills cavity C.

S32, adding liquid medicine: switching the temporary stop member 16 to the second state, and in a state when the medication outlet 12 is closed and the second exhaust port 15 is open, injecting liquid medicine into the injection tube 1 through the medication inlet 11, causing the second piston 22 originally located at the first end to move toward the second end under the pushing of the liquid medicine, until the second piston 22 is in tight contact with the first piston 21, and closing the medication inlet 11 and the second exhaust port 15.

FIG. 3 shows the temporary stop member 16 in the first state. When the temporary stop member 16 is switched to the second state, the temporary stop member 16 retracts into the tube body of the injection tube 1 so that the temporary stop member 16 does not protrude from the inner wall of the injection tube 1. Therefore, the second piston 22 can move to a position in contact with the first piston 21, and the first piston 21 can move toward the medication outlet 12 in the subsequent Step S33.

The second piston 22 originally being at the first end means that in the factory state of the liquid medicine supply device, the second piston 22 is located at the position shown in FIG. 3, which can ensure that the liquid medicine added to the injection tube will not accumulate air bubbles. As the liquid medicine is continuously injected, the second piston 22 moves toward the first piston 21 under the pushing of the liquid medicine, the volume of cavity B formed between the second piston 22 and the first piston 21 continuously decreases, and the gas in cavity B is discharged from the second exhaust port 15. When the second piston 22 moves toward a position of the first piston 21, it is blocked and cannot continue to move. At this time, cavity B between the second piston 22 and the first piston 21 disappears; that is, the second piston 22 is in tight contact with the first piston 21.

S33, pushing liquid medicine: the medication outlet 12 is opened, and power fluid is pumped to the injection tube 1 by the volumetric pump 4, causing the first piston 21 and the second piston 22 to move toward the first end under the pushing of the power fluid, thereby pushing the liquid medicine in the injection tube 1 to the infusion tube.

Since cavity B has disappeared, the second piston 22 is in tight contact with the first piston 21, and the side of the second piston 22 away from the first piston 21 and the side of the first piston 21 away from the second piston 22 are filled with liquid medicine and power fluid respectively, in Step S33, the amount of power fluid pumped into the injection tube 1 by the volumetric pump 4 is equal to the amount of liquid medicine flowing out of the medication outlet 12.

In specific practical applications, the power fluid can be hydraulic oil or purified water, and the volumetric pump 4 can be a diaphragm pump. As shown in FIG. 1, in this embodiment, the volume change driver 41 of the volumetric pump 4 is provided with an elastically deformable diaphragm. Specifically, the diaphragm can be driven to deform through the inverse piezoelectric effect, etc., and the diaphragm pump pumps out a fixed volume of liquid every time it receives a control signal. In order to improve control accuracy, the liquid inlet 43 and the liquid outlet 42 of the volumetric pump 4 are generally provided with check valves.

In a desired embodiment, the liquid medicine supply device comprises a controller electrically connected to the volumetric pump 4, and the controller is provided with a timing module, which facilitates pumping power fluid at predetermined time intervals. In order to facilitate understanding of the current state of the liquid medicine supply device, a pressure sensor 3 can be provided in the first tube section to determine whether the working pressure is normal according to the pressure value detected by the pressure sensor 3. Moreover, in the case where the liquid medicine supply device provided by Embodiment 2 has completed the addition of power fluid in advance at the factory, that is, the first tube section is filled with power fluid in advance, it can also be determined whether the liquid medicine is fully added according to the pressure value detected by the pressure sensor 3 in the step of adding liquid medicine, that is, whether the second piston 22 has come into contact with the first piston 21.

In a desired embodiment, a sealing member for closing the first exhaust port 14 is provided at the first exhaust port 14, and the sealing member is made of a material capable of absorbing the power fluid and swelling, which can achieve automatic closing of the first exhaust port 14. It should be noted that the swelling material used in the present application is a known material. The form of the limiting structure 13 can have various choices; for example, the limiting structure 13 can be configured as an annular rib on the inner wall of the injection tube 1, or configured as a plurality of protrusions distributed circumferentially on the inner wall of the injection tube 1.

In order to realize the closing and opening of the medication outlet 12, a switching valve can be provided at the medication outlet 12, and this switching valve can specifically be an electronically controlled valve. In addition, the closing and opening of the medication outlet 12 can also be realized by a liquid stopping clamp manually controlled. For example, a hose is connected to the medication outlet 12, a liquid stopping clamp is provided at an end of the hose close to the liquid outlet 42, and a connector for connecting to the infusion tube is provided at an end of the hose away from the liquid outlet 42. Of course, in the case where a hose is connected to the medication outlet 12, an electronically controlled liquid stopper can also be provided at the end of the hose close to the liquid outlet 42, so as to control the closing and opening of the medication outlet 12 through software.

In this specification, the structures of various parts are described in a progressive manner. The focus of the description of the structure of each part is the difference from the existing structure. The overall and partial structures of the liquid medicine supply device can be obtained by combining the structures of the above multiple parts.

The above description of the disclosed embodiments enables those skilled in the art to implement or use the present application. Various modifications to these embodiments will be apparent to those skilled in the art, and the general principles defined herein can be implemented in other embodiments without departing from the spirit or scope of the present application. Therefore, the present application will not be limited to these embodiments shown herein, but is to be accorded the widest scope consistent with the principles and novel features disclosed herein.

## Claims

1. A liquid medicine supply device, **characterized in that**, comprises:
an injection tube having a first end and a second end opposite to each other, wherein the first end is provided with a medication inlet and a medication outlet, the medication outlet is for connecting to an infusion tube, a limiting structure for stopping a piston is provided inside the injection tube at a position close to the second end, and a first tube section between the limiting structure and the second end is provided with a first exhaust port closable;
a first piston provided within a second tube section, the second tube section is between the limiting structure and the first end;
a liquid storage bag configured to store a power fluid; and
a volumetric pump, wherein a liquid inlet of the volumetric pump is connected to the liquid storage bag through a pipeline, and a liquid outlet of the volumetric pump is connected to the first tube section through a pipeline.

2. The liquid medicine supply device of claim 1, **characterized in that**, further comprises a pressure sensor provided within the first tube section.

3. The liquid medicine supply device of claim 1, **characterized in that**, the volumetric pump is a diaphragm pump.

4. The liquid medicine supply device of claim 1, **characterized in that**, comprises a controller electrically connected to the volumetric pump, and the controller is provided with a timing module.

5. The liquid medicine supply device of claim 1, **characterized in that**, a sealing member for closing the first exhaust port is provided at the first exhaust port, and the sealing member is made of a material capable of absorbing the power fluid and swelling.

6. The liquid medicine supply device of claim 1, **characterized in that**, the medication outlet is connected to a hose, an electronically controlled liquid stopper or a liquid stopping clamp manually controlled is provided at an end of the hose close to the liquid outlet, and a connector for connecting to the infusion tube is provided at an end of the hose away from the liquid outlet.

7. The liquid medicine supply device according to any one of claims 1 to 6, **characterized in that**, further comprises a second piston provided within the second tube section, the second piston is located on a side of the first piston close to the medication outlet, the second tube section is provided with a second exhaust port closable, and a distance from the second exhaust port to the limiting structure matches a thickness of the first piston.

8. The liquid medicine supply device of claim 7, **characterized in that**, further comprises a temporary stop member arranged with the second tube section, and a distance from the temporary stop member to the limiting structure matches the thickness of the first piston;
wherein the temporary stop member at least has a first state and a second state, wherein in the first state, the temporary stop member protrudes from an inner wall of the injection tube, and in the second state, the temporary stop member does not protrude from the inner wall of the injection tube.

9. A use method for a liquid medicine supply device, **characterized in that**, the liquid medicine supply device is the liquid medicine supply device of any one of claims 1 to 6, the use method comprises:
Step A, adding liquid medicine: in a state when the medication outlet is closed and the first exhaust port is open, injecting liquid medicine into the injection tube through the medication inlet, causing the first piston originally located at the first end to move toward a position of the limiting structure under the pushing of the liquid medicine, and closing the medication inlet;
Step B, adding power fluid: in a state when the first exhaust port faces upward, injecting a portion of the power fluid in the liquid storage bag into the injection tube by the volumetric pump, causing the first tube section to be filled with the power fluid, and closing the first exhaust port; and
Step C, pushing liquid medicine: opening the medication outlet, and pumping the power fluid to the injection tube by the volumetric pump, causing the first piston to move toward the first end under the pushing of the power fluid, thereby pushing the liquid medicine in the injection tube to the infusion tube.

10. A use method for a liquid medicine supply device, **characterized in that**, the liquid medicine supply device is the liquid medicine supply device of claim 8, the use method comprises:
Step A, adding power fluid: switching the temporary stop member to the first state to stick the first piston originally located at the limiting structure, and in a state when the first exhaust port faces upward and is open, injecting a portion of the power fluid in the liquid storage bag into the injection tube by the volumetric pump, causing the first tube section to be filled with the power fluid, and closing the first exhaust port;
Step B, adding liquid medicine: switching the temporary stop member to the second state, and in a state when the medication outlet is closed and the second exhaust port is open, injecting liquid medicine into the injection tube through the medication inlet, causing the second piston originally located at the first end to move toward the second end under the pushing of the liquid medicine, until the second piston is in tight contact with the first piston, and closing the medication inlet and the second exhaust port; and
Step C, pushing liquid medicine: opening the medication outlet, and pumping the power fluid to the injection tube by the volumetric pump, causing the first piston and the second piston to move toward the first end under the pushing of the power fluid, thereby pushing the liquid medicine in the injection tube to the infusion tube.
